Europäisches Patentamt

European Patent Office

Office européen des brevets

(19)

(11) Publication number: 0 344 956
A1

# (12) EUROPEAN PATENT APPLICATION

(21) Application number: 89305172.2

(51) Int. Cl.⁴: A61M 5/31

(22) Date of filing: 23.05.89

(30) Priority: 28.05.88 GB 8812793

(43) Date of publication of application:
06.12.89 Bulletin 89/49

(84) Designated Contracting States:
DE ES FR GB IT NL SE

(71) Applicant: NATIONAL RESEARCH
DEVELOPMENT CORPORATION
101 Newington Causeway
London SE1 6BU(GB)

(72) Inventor: Byrne, Phillip Owen
48 Whaggs Lane
Whickham Newcastle upon Tyne NE16
4PQ(GB)
Inventor: Sisson, Penelope Rosemary
12 Mitchell Avenue
Jesmond Newcastle upon Tyne(GB)
Inventor: Ingham, Harry Raymond
53 Linden Way Darras Hall
Ponteland Newcastle upon Tyne(GB)

(74) Representative: Virr, Dennis Austin et al
Urquhart-Dykes & Lord Floor B Milburn
House Dean Street
Newcastle upon Tyne NE1 1LE(GB)

(54) Single-use hypodermic syringe and adaptor therefor.

(57) An adaptor (20; 40; 70; 90) for a hypodermic syringe, devised to render the syringe less readily illicitly re-usable, has an adaptor body (23; 41; 71, 72; 91) with a liquid-flow passage extending through it between a first end for connecting to the syringe and a second end for connecting to a hypodermic needle, and a valve (27; 50; 82, 83; 99), which controls liquid flow through the passage and is irreversibly operable to prevent subsequent liquid flow through the passage. The irreversible operation of the valve may be manual or may be effected automatically by or following discharge of the syringe contents.

Fig. 1

Fig. 7

## Single-use hypodermic syringe and adaptor therefor

The present invention is concerned with hypodermic syringes and in particular with the adaptation of such syringes to inhibit their re-use.

If a hypodermic needle and/or the associated syringe is to be used a second time, then it is necessary to clean and re-sterilise the equipment to avoid transmitting infection to the second patient. In order to avoid the necessity of such re-sterilising, hypodermic equipment has been developed which allows the needle and/or the syringe economically to be discarded after a single use. However syringes discarded in this way may be retrieved for subsequent illicit use, for example for self-administration of drugs, and this illicit use is a serious cause of the spread of infection among users. To reduce or prevent illicit use of hypodermic syringes, syringes have therefore been divised which are intended to be incapable of further use after they have been used legitimately a single time.

Prior "single-use" syringes have proved to be only partly successful. For example, in syringes wherein the plunger is locked in position within the barrel after ejection of its contents, the locking device may often be over-ridden by a determined potential user, in a manner which leaves the syringe in a condition permitting its re-use. Other single-use syringes require the legitimate first user to perform a specific operation to secure the equipment against re-use or only become effective against re-use when the whole of the contents of the barrel have been discharged. Thus such syringes are vulnerable to casual or deliberate misoperation by the legitimate user leaving the equipment in a re-usable condition.

It is an object of the present invention to provide an adaptor by means of which a hypodermic syringe may be rendered less readily illicitly re-usable than in the case of prior such devices. The invention also includes hypodermic syringes which have been adapted in this way.

The syringe adaptor according to the present invention comprises an adaptor body having a liquid-flow passage therethrough extending between a first end of said passage adapted to be placed in liquid communication with a hypodermic syringe and a second end of said passage adapted to be placed in liquid communication with a hypodermic needle, and a valve, disposed to control liquid flow through said passage and irreversibly operable to prevent subsequent liquid flow through said passage

Be means of the adapter, a hypodermic syringe may thus be modified to enable its normal use for delivering an injection for example, and then for the valve to be irreversibly operated to prevent any subsequent use of the syringe.

The invention includes a hypodermic syringe having an adaptor disposed at the discharge end of the syringe barrel and having a liquid-flow passage therethrough extending from the barrel discharge outlet to a second end of said passage adapted to support a hypodermic needle, and a valve, disposed to control liquid flow through said passage and irreversibly operable to prevent subsequent flow through said passage.

Preferably the irreversible operation of the valve is such as to prevent subsequent liquid flow in a direction from the second end of the passage towards the end nearer to the syringe, that is to prevent subsequent filling of the syringe but, less preferably, the valve may be so operable as to prevent subsequent discharge of the contents of the syringe.

The irreversible operation of the valve may be effected manually by the initial user of the syringe, for example as a separate step following complete or partial discharge of the contents of the syringe or as one aspect of putting the valve into a condition whereby the initial or subsequent discharge of the syringe contents may take place. However, it is much preferred that the irreversible operation of the valve to prevent its subsequent use be effected automatically by or following discharge of the syringe contents. In either case, that is where the irreversible operation of the valve is manual or automatic, that operation may follow the very first discharge of the contents of the syringe or, where the syringe is used to perform a function more complicated than simple injection or sampling, the irreversible operation may occur at a later stage in the operational sequence.

The valve may take numerous possible forms. For example, in the manually-operable form of the adaptor, relative linear or rotary movement of two relatively movable parts of the adaptor may cause a valve member to abut irreversibly a valve seat within the adaptor body and thus to prevent liquid flow through the valve. In another manually-operable form of the adaptor, the valve may comprise two valve units, each permitting flow of liquid in only one direction, and relative movement of two said adaptor parts in order to bring one of said valve units into operation (for example, to discharge the syringe contents) may simultaneously irreversibly render the other said valve unit inoperable.

In a particularly preferred form of the invention in which the valve is automatically irreversibly rendered inoperable, successive movements of a valve member towards and away from a valve seat are limited by interengagement between the valve member and the adaptor body and said successive movements are effected by liquid pressure upon the valve member, the last of said successive movements bringing the valve member irreversibly into sealing engagement with the valve seat. The interengagement of the valve member and the adaptor body may comprise one or more projections on one of the valve member and adaptor body extending into one or more corresponding channels on the other of the valve member and adaptor body. A continuous such channel may then define the sequence of possible movement of the valve member from initial assembly of the adaptor to final irreversible sealing of the valve member against the valve seat.

The invention will now be further described and illustrated with reference to the accompanying drawings, which display several preferred embodiments of the syringe adaptor according to the present invention and wherein:-

Fig. 1 is a vertical cross-sectional view of a first embodiment of syringe adaptor, which is automatic in its operation and is illustrated in its condition before use;

Fig. 2 is a view corresponding to Fig. 1, with the valve member in its final position;

Fig. 3 illustrates, to a larger scale and developed on to a flat surface, a continuous channel which is a feature of the adaptor of Figs. 1 and 2;

Fig. 4 is a vertical cross-sectional view of a second embodiment of syringe adaptor, which is manually operated and is illustrated in its condition before use;

Fig. 5 is a view corresponding to Fig. 4, with the valve member in its final position;

Fig. 6 is a cross-sectional view on the line VI-VI of Fig. 5;

Fig. 7 is a vertical cross-sectional view of a third embodiment of syringe adaptor, which is manually operable;

Fig. 8 is a cross-sectional view on the line VIII-VIII of Fig. 7;

Fig. 9 is a cross-sectional view, also on the line VIII-VIII of Fig. 7 but in the opposite direction IX-IX; and

Fig. 10 is a vertical cross-sectional view of a manually-operable fourth embodiment of syringe adaptor.

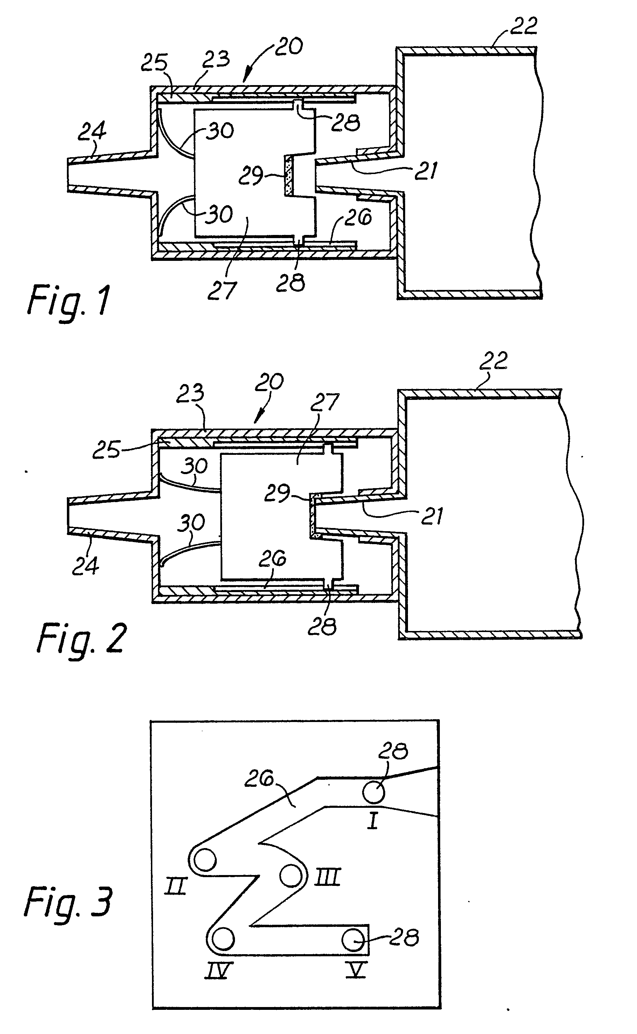

Referring firstly to Figs. 1 and 2, the adaptor 20 illustrated is secured by adhesion or welding to the outlet spigot 21 of the barrel 22 of a conventional hypodermic syringe. The adaptor 20 comprises a cylindrical body 23 provided with an outlet spigot 24 to receive the hub of a hypodermic needle (not shown). Formed integral with the inner surface of the adaptor body 23, or secured thereto as a separate insert, is a cylindrical sleeve 25 into which is moulded a channel or track 26, illustrated in developed form in Fig. 3. Preferably two or more such channels 26 are provided, disposed symmetrically about the cylindrical surface of the sleeve 25.

A generally cylindrical valve member 27 is axially located within the adaptor body 23 and is free to move in an axial direction, within limits defined by radial projections 28 engaging the channels 26. In a circular socket in one end of the valve member 27 is disposed a soft pad 29, aligned with the end of the outlet spigot 21 so as to be able to abut the spigot end and seal it when the projections 28 and channels 26 allow the valve member to move into a sealing position. Two plastic strip springs 30, extending from the other end of the valve member 27 and abutting the end wall of the adaptor body urge the valve member towards the right as illustrated.

When the adaptor is first assembled, the valve member is introduced to the right-hand end of the adaptor body, with the projections 28 entering the channel 26 at position I (Fig. 3). The valve member is advanced until the projections 28 are at position II, from where the springs 30 urge the valve member to the right (position III), but not so far as to be able to seal the syringe outlet spigot 21. In this position, the syringe and adaptor are ready for use.

The syringe barrel may now be filled in the usual way, by retracting the syringe plunger (not shown). Liquid can readily flow past the valve member 27. The pressure of the incoming liquid assists the springs 30 in retaining the valve member in position III. The syringe may now be used to give an injection or to expel its contents for some other purpose and the pressure of the outflowing liquid moves the valve member to position IV. When the ejecting pressure is removed, the valve member is moved by the springs 30 into position V. In this position, illustrated in Fig. 2, the pad 29 closes off the syringe outlet spigot 21.

The syringe and adaptor may now safely be discarded. Any attempt to re-fill the syringe is effectively prevented by the valve member 27 and sealing pad 29, which are retained in place by the springs 30. Action designed to introduce liquid into the syringe via the adaptor simply reinforces the seal. If desired, the projections 28, may, in this final position, be "captured" by depressions in the channel 26.

The sequence of operations permitted to the syringe is controlled throughout by the geometry of the channel 26. Thus by modifying the shape of the channel, the adaptor may be "programmed" to permit a variety of different uses.

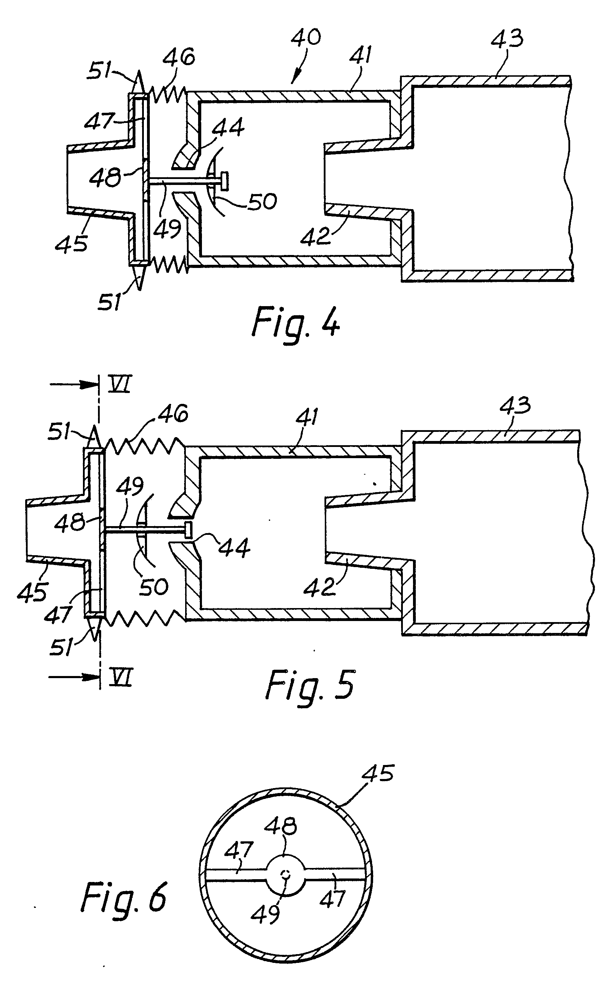

Referring now to Figs. 4 to 6, the illustrated adaptor 40 comprises a cylindrical adaptor body 41, secured in position over the outlet spigot 42 of a syringe barrel 43. The adaptor body 41 has an outlet aperture 44 located at the centre of one end thereof opposite to the spigot 42.

An adaptor outlet spigot unit 45 is aligned with the aperture 44 and is retained in position upon the end of the adaptor 40 by means of a cylindrical bellows 46, which allows the spigot unit 45 to move relative to the adaptor body 40 between the positions illustrated in Figs. 4 and 5 respectively. Radial spokes 47 of the spigot unit 45 support a guard disc 48, from which a valve stem 49 extends axially through the aperture 44. A valve member 50 of soft, deformable plastics material is free to slide along the valve stem 49. The guard disc 48 is designed to prevent illicit access to the valve via the spigot unit.

When the syringe barrel 43 is first to be filled, liquid can be drawn in past the valve and through the adaptor without difficulty. However any attempt to expel the syringe contents causes the valve member 50 to seat against the end of the aperture 44 and seal it. When the syringe is ready for use to administer an injection for example, the operator moves the spigot unit 45 towards the left as illustrated by means of the projections 51, thus expanding the bellows 46 and drawing the flexible valve member 50 through the aperture 44. In this position, the valve member 50 allows liquid to flow from the adaptor out through the aperture 44.

Any subsequent attempt to refill the syringe causes the valve member 50 to seat against the outer end of the aperture 44 and thereby seal the adaptor and syringe against re-use.

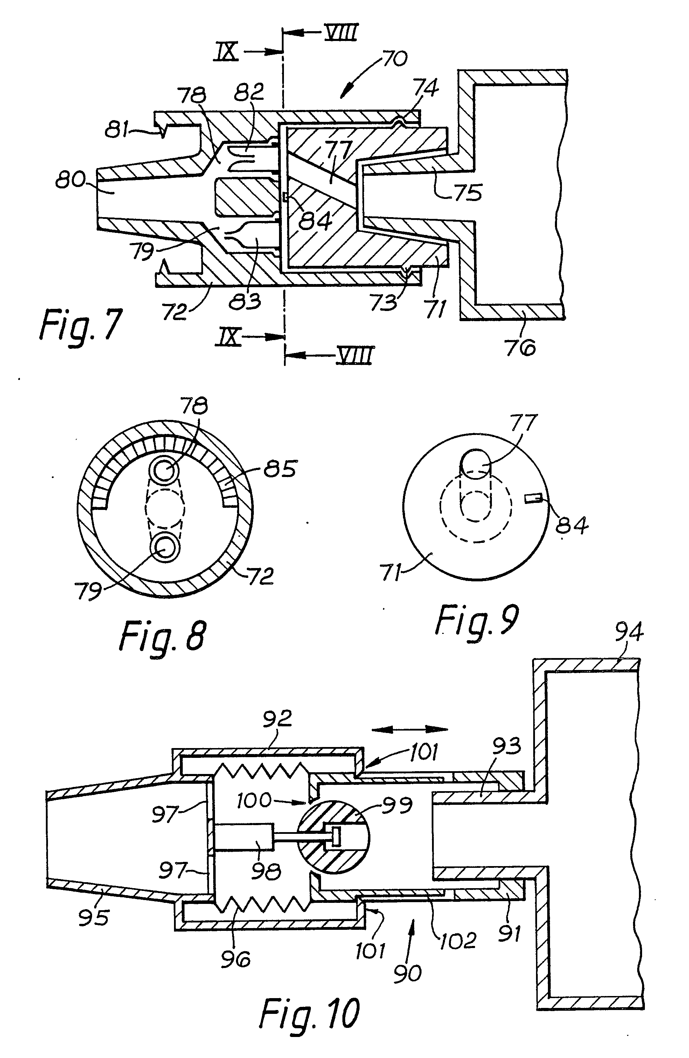

The adaptor 70 illustrated in Figs. 7 to 9 is also manually operated. The adaptor is in the form of two relatively-rotatable parts, namely an inner adaptor part 71 and an outer adaptor part 72, secured together against relative axial movement by an annular projection 73 on the inner part 71 engaging an annular groove 74 on the outer part 72. The inner adaptor part 71 is secured upon the outlet spigot 75 of a conventional syringe barrel 76, or alternatively may be formed integral with the syringe barrel. An inclined liquid passage 77 extends from the syringe outlet spigot 75 through the adaptor part 71.

The outer adaptor part 72 has two parallel liquid passages 78 and 79 which, by rotation of the part 72, can in turn be brought into alignment with the inclined passage 77. The passages 78 and 79 are each in direct communication with an adaptor outlet spigot 80. A luer lock 81 is provided for connection to the housing of a hypodermic needle (not shown).

Within the respective liquid passages 78 and 79 are mounted two oppositely-directed, one-way bunsen valves 82 and 83. Valve 82 permits only inward flow into the adaptor and valve 83 permits only outward flow from the adaptor. Thus the direction of flow of liquid permitted through the adaptor is determined by the relative rotational positions of the two adaptor parts 71 and 72.

Initially, the adaptor part 72 is in the illustrated position. Liquid may therefore be drawn into the syringe barrel via the inlet passage 78 but cannot be expelled from the syringe. In order to administer an injection or to eject the syringe contents for another purpose, the operator must rotate the outer adaptor part 72 through a sufficient angle (180° in the illustrated embodiment) to bring the outlet passage 79 into alignment with the inclined passage 77.

A barb 84 on the adaptor part 71 engages a ratchet track 85 on the part 72 as the two parts are relatively rotated and stops and locks the part 72 against further rotation when the passages 77 and 79 are aligned. The barb and ratchet also ensure together that the rotation cannot be reversed. Thus, when the ejection of liquid from the syringe is completed, refilling of the syringe is prevented by the valve 83.

While, in this embodiment, the illustrated valves 82 and 83 are bunsen valves, other forms of one-way valve, for example simple flap valves of plastics material, may alternatively be used.

Referring now finally to Fig. 10, the illustrated adaptor 90 comprises an adaptor body 91 and an outlet spigot unit 92 partially enclosing and slideable linearly along the body 91. The adaptor is secured over the outlet spigot 93 of the barrel 94 of a conventional hypodermic syringe. The spigot unit 92 has an adaptor outlet spigot 95 aligned with the spigot 93 and is linked to the body 91 by an expandable bellows 96, which forms a liquid seal around the valve. On a radial spider or spokes 97, the spigot unit carries a valve shaft 98, upon which a ball valve member 99 is slidable between limiting positions.

In the illustrated initial condition, the syringe may be filled with liquid by means of the conventional plunger. The valve member 99 is able to lift away from its illustrated position against an annular valve seat 100 and allow liquid into the adaptor. However, liquid cannot be expelled from the syringe because pressure applied to the plunger simply presses the member 99 into sealing engagement with the valve seat 100. In order to deliver an injection, the operator must first slide the spigot unit 92 towards the right as illustrated. Projections 101 on the unit 92 engage tracks 102 on the body 91 and, at the limiting position towards the right, the projections irreversibly enter slots in the tracks and the unit 92 is therefore locked in that position.

Movement of the spigot unit 92 into this position lifts the valve member 99 away from the seat 100 and allows the liquid from the syringe to be expelled. However, subsequent filling of the syringe barrel is prevented by the valve member seating itself, under liquid pressure, against the end of the syringe outlet spigot 93. In this way, illicit further use of the syringe is made impossible.

## Claims

1. An adaptor (20; 40; 70; 90) for a hypodermic syringe, characterised in that the adaptor comprises an adaptor body (23; 41; 71; 72; 91) having a liquid-flow passage therethrough extending between a first end of said passage adapted to be placed in liquid communication with a said hypodermic syringe (22; 43; 76; 94) and a second end of said passage adapted (24; 45; 80, 81; 95) to be placed in liquid communication with a hypodermic needle, and a valve (27; 50; 82, 83; 99), disposed to control liquid flow through said passage and irreversibly operable to prevent subsequent liquid flow through said passage.

2. A syringe adaptor according to claim 1, characterised in that said irreversible operation of the valve prevents subsequent liquid flow from said first end of the liquid-flow passage towards the second end thereof.

3. A syringe adaptor according to claim 1, characterised in that said irreversible operation of the valve prevents subsequent liquid flow from said second end of the liquid-flow passage towards the first end thereof.

4. A syringe adaptor according to claim 3, characterised in that said irreversible operation is effected manually, as a feature of putting the valve into a condition whereby liquid is permitted to flow from the first end of the liquid-flow passage towards the second end thereof.

5. A syringe adaptor according to claim 4, characterised in that it comprises two relatively movable parts (41, 45; 91, 92), relative linear or rotary movement of which to permit said permitted liquid flow causes a valve member (50; 99) to abut a valve seat (44; 100) within the adaptor body (41, 91) and thereby prevent subsequent liquid flow through the valve from the second end towards the first end of the liquid-flow passage.

6. A syringe adaptor according to claim 4, characterised in that the valve comprises two valve units (82, 83), each permitting liquid flow in only one direction, and the adaptor comprises two relatively movable parts (71, 72), relative linear or rotary movement of which to bring one of the valve units (83) into operation irreversibly renders the other valve unit (82) inoperable.

7. A syringe adaptor according to claim 3, characterised in that said irreversible operation of the valve (27) is effected automatically by or following flow of liquid from the first end of the liquid-flow passage towards the second end thereof.

8. A syringe adaptor according to claim 7, characterised in that successive movements of a valve member (27) towards and away from a valve seat (21) are limited by interengagement (26, 28) between the valve member (27) and the adaptor body (23) and are effected by liquid pressure upon the valve member, the last of which successive movements brings the valve member (27) irreversibly into sealing engagement with the valve seat (21).

9. A syringe adaptor according to claim 8, characterised in that the interengagement of the valve member (27) and the adaptor body (23) comprises one or more projections (28) on one of the valve member and adaptor body extending into one or more corresponding channels (26) on the other of the valve member and adaptor body.

10. A hypodermic syringe, characterised by having disposed at the discharge end (21; 42; 75; 93) of the syringe barrel (22; 43; 76; 94) an adaptor (20; 40; 70; 90) as claimed in any of the preceding claims.

Fig. 1

Fig. 2

Fig. 3

*Fig. 4*

*Fig. 5*

*Fig. 6*

Fig. 7

Fig. 8

Fig. 9

Fig. 10

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| Y | DE-A-2 455 631 (WALTER SARSTEDT KUNSTSTOFF-SPRITZGUSSWERK) <br> * Figure 1; page 2, line 12 - page 3, line 3; page 7, line 4 - page 9, line 12 * | 1-4,8-10 | A 61 M 5/31 |
| Y | EP-A-0 268 445 (STERIMATIC HOLDINGS LTD) <br> * Figures 3,4,6,7; column 5, lines 1-8,16-30; column 6, line 53 - column 8, line 7 * | 1-4,8-10 | |
| A | US-A-4 699 614 (GLAZIER) <br> * Figures 1-4 * | 1 | |
| A | WO-A-8 802 640 (STERIMATIC HOLDINGS LTD) <br> * Figures 1-4; page 5, line 7 - page 8, line 4; page 13, lines 15-19 * | 1 | |

TECHNICAL FIELDS SEARCHED (Int. Cl.4)

A 61 M

The present search report has been drawn up for all claims

| Place of search | Date of completion of the search | Examiner |
|---|---|---|
| THE HAGUE | 05-09-1989 | SEDY, R. |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P0401)